# EUROPEAN PATENT APPLICATION

(11) **EP 4 455 268 A1**
(43) Date of publication of application: **30.10.2024**
(21) Application number: 22911275.0
(22) Date of filing: 21.12.2022
(51) Int. Cl.: C12N 5/071, C12M 1/00, C12M 3/00, C12N 5/07, C12N 5/09, C12N 5/10

(54) **METHOD FOR CULTURING THREE-DIMENSIONAL CELL TISSUE**

(30) Priority: 22.12.2021 JP 2021208086; 06.06.2022 JP 2022091422
(71) Applicant: Toppan Holdings Inc., Tokyo 110-0016 (JP)
(72) Inventor: HIRAOKA, Yasuyuki, Tokyo 110-0016 (JP)
(74) Representative: Plasseraud IP
(86) International application number: PCT/JP2022/047103
(87) International publication number: WO 2023/120573

(57) **Abstract**

A method of culturing three-dimensional cellular tissues, including the steps of: mixing cells with a cationic substance, an extracellular matrix component and a polyelectrolyte to obtain a mixture; collecting cells from the obtained mixture to obtain cell aggregates; seeding the cell aggregates in a culture medium stored in a culture vessel; and culturing the seeded cell aggregates without suspension to thereby obtain a three-dimensional cellular tissue.

## Description

### [Technical Field]

The present invention relates to a method of culturing three-dimensional cellular tissues.

The present application is based on and claims the benefits of priorities from Japanese Patent Application No. 2021-208086 filed December 22, 2021 and No. 2022-091422 filed June 6, 2022, the contents of which are incorporated herein by reference.

### [Background Art]

In recent years, the superiority of using three-dimensional cellular tissues organized three-dimensionally over cells grown on a flat plate has been shown not only in regenerative medicine but also in assay systems for drugs that require an environment close to that of a living body. Accordingly, various techniques for constructing three-dimensional cellular tissues in vitro have been developed. For example, a method of forming a cell mass on a surface substrate to which cells cannot adhere, a method of forming a cell mass in a droplet, a method of integrating cells on a permeable membrane, and the like have been developed. In order to maintain such cell organization, an extracellular matrix (also referred to as ECM) such as collagen produced by the living body itself is required for cell-cell binding and scaffold formation. Therefore, when constructing cellular tissue artificially, externally adding an ECM has been considered.

PTL 1 discloses a method of culturing cells isolated by enzyme treatment or the like as three-dimensional aggregates after bringing the cells into contact with collagen or the like, which is a typical ECM and a water-soluble polymer having a cytoprotective effect. According to this method, a water-soluble polymer gel is formed around the cells during culture. PTL 2 discloses a method of constructing a three-dimensional tissue by preparing cell sheets using a culture dish having poly(N-isopropylacrylamide) (PIPAAm), which is a temperature-responsive resin, immobilized on the surface, and laminating the prepared cell sheets.

The inventors have conducted research on methods of preparing three-dimensional cellular tissue by mixing cells with a cationic substance, an extracellular matrix component and a polyelectrolyte, and culturing the mixture (for example, see PTL 3).

### [Citation List]

### [Patent Literature]

PTL 1: JP 2824081 B
PTL 2: WO 2002/008387 A
PTL 3: JP 6427836 B
PTL 4: WO 2021/100709 A

### [Non-Patent Literature]

NPL 1: Nishiguchi A et al., "Cell-Cell Crosslinking by Bio-Molecular Recognition of Heparin-Based Layer-by-Layer Nanofilms", Macromol Biosci., vol. 15, 312-317, 2015.
NPL 2: Kukla et al., Microscale Collagen and Fibroblast Interactions Enhance Primary Human Hepatocyte Functions in Three-Dimensional Models, Gene Expr., 20(1), 1-18, 2020.

### [Summary of Invention]

### [Technical Problem]

When a three-dimensional cellular tissue is prepared by the method described in PTL 3, cells are seeded over the entire seeding surface of a culture vessel to form a three-dimensional tissue. Accordingly, the amount of cells to be seeded needs to be increased as the bottom area of the vessel increases. This increases the amount of cells per unit volume of the culture medium, resulting in nutrient depletion in the culture medium relative to the total amount of cells.

Therefore, the present invention has been made to provide a method of culturing three-dimensional cellular tissues capable of reducing the number of cells to be seeded to produce a three-dimensional cellular tissue, and provide a technique for producing integrated three-dimensional cellular tissue at a single location.

### [Solution to Problem]

The inventors have found that, in a method of preparing three-dimensional cellular tissue by mixing cells with a cationic substance, an extracellular matrix component and a polyelectrolyte and culturing the mixture, a three-dimensional cellular tissue can be produced with a small number of cells by seeding the cells in a culture medium and then culturing the seeded cells without suspending them in the entire culture medium, and thus completed the present invention. That is, the present invention includes the following aspects.

[1] A method of culturing three-dimensional cellular tissues, including the steps of: mixing cells with a cationic substance, an extracellular matrix component and a polyelectrolyte to obtain a mixture; collecting cells from the obtained mixture to obtain cell aggregates; seeding the cell aggregates in a culture medium stored in a culture vessel; and culturing the seeded cell aggregates without suspension to thereby obtain a three-dimensional cellular tissue.
[2] The method of culturing three-dimensional cellular tissues according to [1], wherein in the step of seeding the cell aggregates in a culture medium stored in a culture vessel, 3×10⁴ to 1×10⁷ cells are seeded per mL of culture medium.
[3] The method of culturing three-dimensional cellular tissues according to [1] or [2], wherein the culture vessel is an adhesive culture vessel.
[4] The method of culturing three-dimensional cellular tissues according to any one of [1] to [3], wherein the extracellular matrix component is selected from the group consisting of collagen, laminin, fibronectin, vitronectin, elastin, tenascin, entactin, fibrillin, proteoglycan, and combinations thereof, and the polyelectrolyte is selected from the group consisting of glycosaminoglycans, dextran sulfate, rhamnan sulfate, fucoidan, carrageenan, polystyrene sulfonic acid, polyacrylamido-2-methylpropanesulfonic acid, polyacrylic acid, and combinations thereof.
[5] The method of culturing three-dimensional cellular tissues according to any one of [1] to [4], wherein a concentration of the extracellular matrix component in the mixture is 0.01 mg/mL or greater and less than 1.0 mg/mL, and a concentration of the polyelectrolyte in the mixture is 0.01 mg/mL or greater and less than 10 mg/mL.

The present invention includes other aspects as follows.
[6] A method of producing three-dimensional cellular tissues, including the step of: suspending a population of cells containing second cells in a solution containing a cationic substance, an extracellular matrix component and a polyelectrolyte to obtain a mixture; collecting cells from the obtained mixture to obtain cell aggregates; placing the cell aggregates in contact with a first cellular tissue containing first cells placed in a culture medium; and culturing the cell aggregates and the first cellular tissue without suspension to cause the cell aggregates to form a second cellular tissue, whereby a three-dimensional cellular tissue is formed, in which the second cellular tissue is in contact with the first cellular tissue.
[7] The production method according to [6], wherein the first cells contain vascular endothelial cells, and the second cells contain hepatocytes.
[8] A three-dimensional cellular tissue containing a first cellular tissue containing first cells and a second cellular tissue containing second cells, the second cellular tissue being in contact with the first cellular tissue.
[9] The three-dimensional cellular tissue according to [8], wherein the first cells contain vascular endothelial cells, and the second cells contain hepatocytes.

### [Advantageous Effects of Invention]

According to the present invention, the number of cells to be seeded to produce a three-dimensional cellular tissue can be reduced, and a technique for producing integrated three-dimensional cellular tissue at a single location can be provided.

### [Brief Description of Drawings]

Fig. 1(a) is a schematic diagram illustrating an experimental example according to the related art, and Fig. 1(b) shows fluorescence microscopy images showing the results of the experimental example according to the related art.
Fig. 2(a) is a schematic diagram illustrating a comparative experimental example according to the related art. Fig. 2(b) shows fluorescence microscopy images showing the results of the comparative experimental example according to the related art.
Fig. 3(a) to Fig. 3(c) are graphs showing the results of experimental examples according to the related art.
Fig. 4 shows microscopy images of three-dimensional cellular tissues in Experimental Example 1.
Fig. 5 shows microscopy images of three-dimensional cellular tissues in Experimental Example 2.
Fig. 6 shows microscopy images of three-dimensional cellular tissues in Experimental Example 3.
Fig. 7 shows microscopy images of three-dimensional cellular tissues in Experimental Example 4.
Fig. 8 shows microscopy images of three-dimensional cellular tissues immunohistochemically stained in Experimental Example 5.
Fig. 9 is a fluorescence microscopy image showing the result of Experimental Example 6.
Fig. 10 is a fluorescence microscopy image showing the result of Experimental Example 7.

### [Description of Embodiments]

There is no particular restriction on the form of three-dimensional cellular tissues to which the present invention is applied. For example, the present invention can be applied to three-dimensional cellular tissues formed by culturing cells in a vessel such as a cell culture insert, three-dimensional cellular tissues formed by culturing cells in a scaffold of a natural biopolymer such as collagen or a synthetic polymer, cell aggregates (also referred to as spheroids), sheet-like cell structures, and the like. In the case of three-dimensional cellular tissues having a laminate structure composed of two or more cell layers (for example, sheet-like cell structures), in particular, there is a risk that the originally formed layer configuration may collapse if the tissue becomes thinner due to a decrease in cellular tissues. However, the present invention can suppress such a risk, so it can be more effectively adopted.

The term "three-dimensional cellular tissue" as used herein refers to a three-dimensional aggregate at least containing one type of cells. Cells inside the three-dimensional cellular tissue adhere to each other. Examples of the three-dimensional cellular tissue constructed in the present invention include, but are not limited to, biological tissues such as skin, hair, bone, cartilage, tooth, cornea, blood vessel, lymphatic vessel, heart, liver, pancreas, nerve and esophagus, and solid cancer models (for example, stomach cancer, esophageal cancer, bowel cancer, colon cancer, rectal cancer, pancreatic cancer, breast cancer, ovarian cancer, prostate cancer, renal cell cancer and liver cancer). The three-dimensional cellular tissues can be produced by the production method described below.

Examples of the sheet-like cell structure include those produced by culturing cells in monolayer in a culture vessel, removing the cultured cells from the culture vessel and laminating them on other monolayer cultured cells, and those produced by the method of producing three-dimensional cellular tissues described below.

The term "cell aggregate" as used herein refers to a population of cells. The cell aggregate includes three-dimensional cellular tissues, two-dimensional cellular tissues, and cell precipitates obtained by centrifugation, filtration, or the like. The two-dimensional cellular tissues refer to tissues formed by approximately one layer of cells adhering to a cell culture substrate. The two-dimensional cellular tissues can be produced by, for example, culturing adherent cells in a culture vessel by a conventional method. In an embodiment, the cell aggregate is in the form of a slurry-like viscous body. The "slurry-like viscous body" refers to a gel-like cell aggregate as described in NPL 1.

### <First Embodiment>

### [Culture Method]

The first embodiment is directed to a method of culturing three-dimensional cellular tissues, including the steps of: (A) mixing cells with a cationic substance, an extracellular matrix component and a polyelectrolyte to obtain a mixture; (B) collecting cells from the obtained mixture to obtain cell aggregates; (C) seeding the cell aggregates in a culture medium stored in a culture vessel; and (D) culturing the seeded cell aggregates without suspension to thereby obtain a three-dimensional cellular tissue.

As used herein, unless otherwise specified, the term "seeding" means first seeding cells as cell aggregates in a culture medium before a three-dimensional cellular tissue is formed, and does not mean seeding throughout the whole process including a step of exchanging the culture medium after the three-dimensional cellular tissue is formed.

As will be described later in the examples, three-dimensional cultured tissues can be constructed while reducing the number of cells to be seeded to produce a three-dimensional cellular tissue by mixing cells with a cationic substance, an extracellular matrix component and a polyelectrolyte to obtain a mixture, collecting cells from the obtained mixture to obtain cell aggregates, seeding the obtained cell aggregates in a culture medium stored in a culture vessel, and culturing the cells without suspension.

In step (A) of the method of producing three-dimensional cellular tissues, cells are mixed with a cationic substance, an extracellular matrix component and a polyelectrolyte. By forming cell aggregates from the cell mixture in step (B), a three-dimensional cellular tissue with fewer large voids therein can be obtained. Since the obtained three-dimensional cellular tissue is comparatively stable, it can be cultured for at least several days, and is unlikely to be damaged during medium exchange.

In step (B) of the method of producing three-dimensional cellular tissues, cells are collected from the obtained mixture to obtain cell aggregates. For example, as step (B), a step (B'-0) of placing the mixture on a substrate to obtain cell aggregates or step (B'-1) of removing a liquid part from the obtained mixture to obtain cell aggregates may be performed, or in addition to step (B'-1), a step (B'-2) of suspending cell aggregates in a solution may be performed. When steps (B'-1) and (B'-2) are performed, step (C) may be replaced with a step (C') of seeding the obtained suspension in a culture medium stored in a culture vessel. The solution in (B'-2) is not particularly limited as long as it does not adversely affect the growth of cells and formation of a three-dimensional cellular tissue. For example, a cell culture medium, a buffer solution, or the like suitable for the cells constituting the cell aggregates can be used. Desired tissues can be obtained by performing the above steps (A) to (D), but more homogeneous tissues can be obtained by performing steps (B'-1) and (B'-2) as step (B) and performing step (C') as step (C).

In the method of producing three-dimensional cellular tissues, mixing cells with a cationic substance, a polyelectrolyte and an extracellular matrix component, that is, step (A) may be performed in a suitable vessel such as a dish, tube, flask, bottle or plate. Suspension in step (B'-2) may also be performed in a suitable vessel such as a dish, tube, flask, bottle or plate.

### (Cationic Substance)

As the cationic substance in the method of producing three-dimensional cellular tissues, any positively charged substance can be used as long as it does not adversely affect the growth of cells and formation of cell aggregates. Examples of the cationic substance include, but are not limited to, cationic buffer solutions such as tris-HCl buffer solution, tris-maleic acid buffer solution, bis-tris buffer solution and HEPES; ethanolamine, diethanolamine, triethanolamine, polyvinylamine, polyallylamine, polylysine, polyhistidine and polyarginine. In a preferred embodiment, the cationic substance used in the present invention is a cationic buffer solution. In a more preferred embodiment, the cationic substance used in the present invention is a tris-HCl buffer solution.

The concentration of the cationic substance in the method of producing three-dimensional cellular tissues is not particularly limited as long as it does not adversely affect the growth of cells and formation of cell aggregate. The concentration of the cationic substance used in the present embodiment is preferably 10 mM to 200 mM. For example, the concentration of the cationic substance used in the present embodiment is more preferably 20 mM to 180 mM, still more preferably 40 mM to 160 mM, still more preferably 60 mM to 140 mM, and still more preferably 80 mM to 120 mM. The concentration of the cationic substance used in the present embodiment is still more preferably 100 mM.

When a cationic buffer solution is used as the cationic substance in the method of producing three-dimensional cellular tissues, the pH of the cationic buffer solution is not particularly limited as long as it does not adversely affect the growth of cells and formation of cell aggregates. The pH of the cationic buffer solution used in the present embodiment is preferably 6.0 to 8.0. For example, the pH of the cationic buffer solution used in the present embodiment may be 7.0, 7.1, 7.2, 7.3, 7.4, 7.5, 7.6, 7.7, 7.8, 7.9 or 8.0. The pH of the cationic buffer solution used in the present embodiment is more preferably 7.2 to 7.6. The pH of the cationic buffer solution used in the present embodiment is still more preferably 7.4.

### (Polyelectrolyte)

The term "polyelectrolyte" as used herein refers to a polymer having a dissociable functional group in the polymer chain. As the polyelectrolyte used in the present embodiment, any polyelectrolyte can be used as long as it does not adversely affect the growth of cells and formation of cell aggregate. Examples of the polyelectrolyte include, but are not limited to, glycosaminoglycans such as heparin, chondroitin sulfate (e.g., chondroitin 4-sulfate and chondroitin 6-sulfate), heparan sulfate, dermatan sulfate, keratan sulfate and hyaluronic acid; dextran sulfate, rhamnan sulfate, fucoidan, carrageenan, polystyrene sulfonic acid, polyacrylamido-2-methylpropanesulfonic acid, polyacrylic acid, and the like. These polyelectrolytes may be used singly or in combination of two or more. The polyelectrolyte used in the present embodiment is preferably a glycosaminoglycan. Further, the polyelectrolyte used in the present embodiment is more preferably heparin, dextran sulfate, chondroitin sulfate, or dermatan sulfate. The polyelectrolyte used in the present embodiment is still more preferably heparin. Derivatives of the above polyelectrolytes may also be used as long as they do not adversely affect the growth of cells and formation of cell aggregates.

The concentration of the polyelectrolyte in the method of producing three-dimensional cellular tissues is not particularly limited as long as it does not adversely affect the growth of cells and formation of cell aggregate. The concentration of the polyelectrolyte used in the present embodiment is preferably greater than 0 mg/mL and less than 10.0 mg/mL. The concentration of the polyelectrolyte used in the present embodiment is more preferably 0.01 mg/mL or greater and 5.0 mg/mL or less, and still more preferably 0.05 mg/mL or greater and 3.0 mg/mL or less. For example, the concentration of the polyelectrolyte used in the present embodiment may be 0.01, 0.025, 0.05, 0.075, or 0.1, 0.3, 0.5 or 1.0 mg/mL. The concentration of the polyelectrolyte used in the present embodiment is still more preferably 0.1 mg/mL or greater and 1.0 mg/mL or less. The concentration of the polyelectrolyte used in the present embodiment is still more preferably 0.05 mg/mL.

In the present embodiment, the polyelectrolyte may be dissolved in an appropriate solvent before use. Examples of the solvent include, but are not limited to, water and buffer solutions. When a cationic buffer solution is used as the above cationic substance, the polyelectrolyte may be dissolved in a cationic buffer solution before use.

### (Extracellular Matrix Component)

As the extracellular matrix component in the method of producing three-dimensional cellular tissues, any component that constitutes an extracellular matrix can be used as long as it does not adversely affect the growth of cells and formation of cell aggregates. Examples of the extracellular matrix component include, but are not limited to, collagen, laminin, fibronectin, vitronectin, elastin, tenascin, entactin, fibrillin, proteoglycan, and the like. These extracellular matrix components may be used singly or in combination thereof.

Examples of the proteoglycan include, but are not limited to, chondroitin sulfate proteoglycan, heparan sulfate proteoglycan, keratan sulfate proteoglycan and dermatan sulfate proteoglycan. The extracellular matrix component used in the present embodiment is preferably at least one of collagen, laminin and fibronectin; and is more preferably collagen. Modified forms and variants of the extracellular matrix components mentioned above may also be used as long as they do not adversely affect the growth of cells and formation of cell aggregates.

The concentration of the extracellular matrix component is not particularly limited as long as it does not adversely affect the growth of cells and formation of cell aggregates. The concentration of the extracellular matrix component used in the present embodiment is preferably greater than 0 mg/mL and less than 1.0 mg/mL. The concentration of the extracellular matrix component used in the present embodiment is more preferably 0.01 mg/mL or greater and 1.0 mg/mL or less, and still more preferably 0.025 mg/mL or greater and 0.5 mg/mL or less. For example, the concentration of the extracellular matrix component may be 0.01, 0.025, 0.05, 0.075, 0.1, 0.2, 0.3, 0.4 or 0.5 mg/mL. The concentration of the extracellular matrix component used in the present embodiment is still more preferably 0.05 mg/mL or greater and 0.3 mg/mL or less. The concentration of the extracellular matrix component used in the present embodiment is still more preferably 0.05 mg/mL. In the present embodiment, the extracellular matrix component may be dissolved in an appropriate solvent before use. Examples of the solvent include, but are not limited to, water, buffer solutions and acetic acid. In the present embodiment, the extracellular matrix component is preferably dissolved in a buffer solution or acetic acid.

In the method of producing three-dimensional cellular tissues, the mixing ratio between the polyelectrolyte and the extracellular matrix component is preferably 1:2 to 2:1. The mixing ratio between the polyelectrolyte and the extracellular matrix component used in the present embodiment is more preferably 1:1.5 to 1.5:1. The mixing ratio between the polyelectrolyte and the extracellular matrix component used in the present embodiment is still more preferably 1:1.

Examples of the cells used in the method of producing three-dimensional cellular tissues include, but are not limited to, cells derived from humans or animals such as monkeys, dogs, cats, rabbits, pigs, cows, mice, and rats. Also, the site of origin of the cells is not particularly limited, and the cells may be somatic cells such as those derived from bone, muscle, viscera, nerve, brain, skin and blood, or may be reproductive cells. Moreover, the cells used in the method according to the present embodiment may be induced pluripotent stem cells (also referred to as iPS cells) or embryonic stem cells (also referred to as ES cells). Alternatively, cultured cells such as primary cultured cells, subcultured cells and cell lines may be used. One type of cells or a plurality of types of cells may be used. Examples of the cells used in the method according to the present embodiment include, but are not limited to, nerve cells, dendritic cells, immune cells, vascular endothelial cells, lymphatic endothelial cells, fibroblasts, cancer cells such as liver cancer cells, epithelial cells, cardiac muscle cells, liver cells, pancreatic islet cells, tissue stem cells, smooth muscle cells, and the like.

Since the three-dimensional cellular tissue of the present embodiment is a tissue that imitates the functions of in vivo tissue, the cells used in the method of producing three-dimensional cellular tissues are preferably cells other than immortalized cells.

The three-dimensional cellular tissue obtained by the method of producing three-dimensional cellular tissues may include one type of cells or a plurality of types of cells. In the present embodiment, cells included in the three-dimensional cellular tissue are selected from the group consisting of neuronal cells, dendritic cells, immune cells, vascular endothelial cells, lymphatic endothelial cells, fibroblasts, cancer cells, epithelial cells, myocardial cells, hepatocytes, pancreatic islet cells, tissue stem cells and smooth muscle cells. Further, the three-dimensional cellular tissue obtained by the method according to the present embodiment may include a vasculature structure. The "vasculature structure" refers to a network structure, such as a blood vessel network or a lymphatic network, in living tissues.

In the method of producing three-dimensional cellular tissues, as a means for collecting the cells in step (B'-0) or step (B'-1), methods known to those skilled in the art can be used. For example, the cells may be collected by centrifugation, magnetic separation or filtration. The liquid part may be removed. The centrifugation conditions are not particularly limited as long as they do not adversely affect the growth of cells and formation of cell aggregates. For example, a microtube or a cell culture insert containing the mixture may be subjected to centrifugation at room temperature (e.g., 25°C) and 400 to 1,000 × g for 2 minute to separate a liquid part from a cell aggregate, thereby collecting cells. Alternatively, the liquid part may be removed after the cells are collected by spontaneous sedimentation. The cell precipitate in the cell aggregate in step (B-0) or (B'-1) may be in the form of a laminate.

The solution used in step (B'-2) in the method of producing three-dimensional cellular tissues is not particularly limited as long as it does not adversely affect the growth of cells and formation of cell aggregate. For example, a cell culture medium or a buffer solution suitable for the cells to be used is used.

The culture vessel used in step (C) in the method of producing three-dimensional cellular tissues may be a vessel having a material and a shape commonly used in cell or microorganism culture. Examples of the material of the culture vessel include, but are not limited to, glass, stainless steel and plastics such as polystyrene having no or low cytotoxicity. Examples of the culture vessel include, but are not limited to, a dish, tube, flask, bottle, plate, and the like. The culture vessel is preferably a vessel having a permeable membrane, for example, a material that does not allow cells in the liquid to pass through but allows the liquid to pass through. Examples of the vessel having a permeable membrane include, but are not limited to, cell culture inserts, such as Transwell (registered trademark) insert, Netwell insert, Falcon (registered trademark) cell culture insert and Millicell (registered trademark) cell culture insert.

The culture vessel may be an adhesive culture vessel or a non-adhesive culture vessel, but an adhesive culture vessel is preferred. Using an adhesive culture vessel enables production of three-dimensional cellular tissues with cells adhering to the vessel, and in this method, in which the cells are immobilized in the vessel, the cells are likely to be integrated locally in the culture medium without dispersing. Furthermore, using an adhesive culture vessel facilitates post-processing operations such as culture medium exchange, evaluation and observation. Examples of the adhesive culture vessel include, but are not limited to, typical polystyrene culture vessels, and culture vessels coated with polymers or the like that promote cell adhesion.

In the method of producing three-dimensional cellular tissues, a substance for suppressing deformation of the constructed three-dimensional cellular tissue (for example, tissue contraction, peeling at the tissue edge, or the like) is used. Examples of such a substance include, but are not limited to, Y-27632, which is a selective ROCK (Rho-associated coiled-coil forming kinase/Rho binding kinase) inhibitor. In the present embodiment, step (D) is performed in the presence of the above substance. Culturing cell aggregates in the presence of the above substance can prevent contraction of the constructed three-dimensional cellular tissue. As a result, the constructed tissue can be prevented from detaching from the culture vessel. For example, such a substance may be further mixed in step (A). Alternatively, such a substance may be further added to the solution used in step (B'-2). Alternatively, such a substance may be further added to the culture medium when culturing cells in step (D).

In step (D) of the method of producing three-dimensional cellular tissue, cell culture can be performed under culture conditions suitable for the cells to be cultured. Those skilled in the art can select a suitable medium depending on the cell type and desired function. Examples of the cell culture medium include, but are not limited to, media such as D-MEM, E-MEM, MEMα, RPMI-1640, Mccoy'5a and Ham's F-12, and media obtained by adding CS (also called bovine serum), FBS (also called fetal bovine serum) or HBS (also called fetal horse serum) to the above media so that the serum content is about 1% to 20% by volume. Conditions such as the temperature of the culture environment and the atmospheric composition can also be easily determined by those skilled in the art.

The number of cells in the three-dimensional cellular tissue may be determined by the number of cells seeded when producing the three-dimensional cellular tissue. Further, the number of cells in the three-dimensional cellular tissue may be the number of living cells. The number of living tissues in the three-dimensional cellular tissue may be 1.5×10⁶ or greater.

In step (C), the number of cells to be seeded is not particularly limited as long as a three-dimensional cellular tissue can be produced, but is preferably 3×10⁴ to 1×10⁷ per mL of culture medium, more preferably 3×10⁴ to 9×10⁶ per mL of culture medium, still more preferably 3×10⁴ to 8×10⁶ per mL of culture medium, still more preferably 3×10⁴ to 7×10⁶ per mL of culture medium, still more preferably 3×10⁴ to 6×10⁶ per mL of culture medium, still more preferably 3×10⁴ to 5×10⁶ per mL of culture medium, still more preferably 3×10⁴ to 4×10⁶ per mL of culture medium, still more preferably 3×10⁴ to 3×10⁶ per mL of culture medium, still more preferably 3×10⁴ to 2×10⁶ per mL of culture medium, and still more preferably 3×10⁴ to 1×10⁶ per mL of culture medium. Further, in step (C), the "number of cells to be seeded" refers to the number of cells per culture medium volume when cells are first seeded in a culture medium before a three-dimensional cellular tissue is formed.

In step (C), the cell aggregates may be seeded in a culture medium by dropping the cell aggregates onto a surface of a culture medium, injecting the cell aggregates into a culture medium, or by forming a culture medium as a liquid droplet and injecting the cell aggregate into the liquid droplet. The cell aggregates are seeded in a state of being integrated locally without dispersing in the culture medium. More preferably, the cell aggregates are seeded in a state of adhering and being integrated locally on the side surface or bottom of the culture vessel without dispersing in the culture medium.

There is no particular restriction on the volume of culture medium initially stored in the culture vessel before a three-dimensional cellular tissue is formed, but reducing the volume of the culture medium can reduce the number of cells first seeded in the culture medium stored in the culture vessel. The volume of culture medium is determined as appropriate depending on the size of the culture vessel used. Further, the volume of culture medium in culture medium exchange after a three-dimensional cellular tissue is formed is not particularly limited and determined as appropriate.

As will be described later in the examples, in step (D), the cell aggregates seeded in step (C) are cultured without suspension. The "culturing the cell aggregates without suspension" means culturing the cell aggregates in a locally dense manner without dispersing substantially uniformly in a culture medium, and preferably means culturing the cell aggregates in a state of adhering to the side surface or bottom of the culture vessel in a locally dense manner. Even when the seeded cell aggregates are cultured without suspension, typical cells naturally disperse in a culture medium and are difficult to form a three-dimensional cellular tissue, and in addition, the cells are integrated two dimensionally. Since this locally induces a high confluent state, which should be avoided in two-dimensional planar culture, the step of culturing without suspension is not suitable for general cell culture. On the other hand, according to the method of producing three-dimensional cellular tissues of the present embodiment, which uses cells that have been subjected to a series of treatments using a cationic substance, an extracellular matrix component and a polyelectrolyte, the cells do not disperse but are integrated locally in the culture medium, increasing the density of cells. Therefore, a three-dimensional cellular tissue can be produced with a reduced number of seeded cells.

When seeding a cell suspension, that is, when steps (B'-1) and (B'-2) are performed as step (B), the density of cells in the cell suspension is not particularly limited, but is preferably 2×10⁶ to 1×10⁸/mL, more preferably 2×10⁶ to 9×10⁷/mL, still more preferably 2×10⁶ to 8×10⁷/mL, still more preferably 2×10⁶ to 7×10⁷/mL, still more preferably 2×10⁶ to 6×10⁷/mL, still more preferably 2×10⁶ to 5×10⁷/mL, still more preferably 2×10⁶ to 4×10⁷/mL, still more preferably 2×10⁶ to 3×10⁷/mL, still more preferably 2×10⁶ to 2×10⁷/mL, still more preferably 2×10⁶ to 1×10⁷/mL, still more preferably 2.5×10⁶ to 1×10⁸/mL, still more preferably 2.5×10⁶ to 9×10⁷/mL, still more preferably 2.5×10⁶ to 8×10⁷/mL, still more preferably 2.5×10⁶ to 7×10⁷/mL, still more preferably 2.5×10⁶ to 6×10⁷/mL, still more preferably 2.5×10⁶ to 5×10⁷/mL, still more preferably 2.5×10⁶ to 4×10⁷/mL, still more preferably 2.5×10⁶ to 3×10⁷/mL, still more preferably 2.5×10⁶ to 2×10⁷/mL, and still more preferably 2.5×10⁶ to 1×10⁷/mL.

The cell aggregates, or the suspended cells when the cell aggregates are suspended, are preferably seeded to be integrated locally at a cell density of 1,000/mm² or greater, more preferably 10,000/mm² or greater, still more preferably 20,000/mm² or greater, and still more preferably 25,000/mm² or greater. Further, the cells can be seeded at a cell density of, for example, 1,000,000/mm² or less, preferably 500,000/mm² or less, more preferably 200,000/mm² or less, and still more preferably 100,000/mm² or less. By performing the steps of suspending the cell aggregates in a solution and precipitating the cells, a more homogeneous three-dimensional cellular tissue can be obtained.

Steps (A) to (C) or (A) to (C') in the method of producing three-dimensional cellular tissues can be repeated to laminate the cell aggregates. Thus, a three-dimensional cellular tissue having a plurality of layers can be constructed. In this case, a plurality of types of cells may be used to construct three-dimensional cellular tissue composed of different types of cells. According to the method of producing three-dimensional cellular tissues, the constructed three-dimensional cellular tissue may have a thickness of about 5 µm to 300 µm, about 400 µm, or about 500 µm. The thickness is preferably 60 µm or greater, more preferably 200 µm or greater, and still more preferably 250 µm or greater. Steps (A) to (C) or (A) to (C') in the above method of the present invention can be repeated to obtain a three-dimensional cellular tissue with fewer large voids therein even when the tissue has a large thickness of, for example, 150 µm to 500 µm. In the present embodiment, the number of cell layers in the constructed three-dimensional cellular tissue is preferably about 1 to 100 layers, and more preferably about 10 to 100 layers. The cell layers refer to layers composed of different layers in which the cell nuclei do not overlap each other in the weight or height direction of the cells, when observed at a magnification at which cell nuclei can be recognized in a section image of the cross-section of the three-dimensional cellular tissue in the thickness direction, that is, at which the entire thickness of the stained section is included in the field of view. In this case, the field of view has at least 200 µm in a (horizontal) direction perpendicular to the weight. In the present embodiment, a section image of the cross-section of the three-dimensional cellular tissue in the thickness direction is observed at 100 to 200x magnification.

The three-dimensional cellular tissue obtained by repeating steps (A) to (C) or (A) to (C') of the method of producing three-dimensional cellular tissues has a smaller number of cell layers per unit thickness than the three-dimensional cellular tissue obtained by a conventional cell lamination method. In the method of producing three-dimensional cellular tissues, the constructed three-dimensional cellular tissue is obtained in vitro. Further, the three-dimensional cellular tissue according to the method of producing three-dimensional cellular tissues contains cells and an extracellular matrix component, and the number of cell layers per 10 µm thickness is 2.8 or less, preferably 2.5 or less, and more preferably 2.2 or less. In the method of producing three-dimensional cellular tissues, in a region of the obtained three-dimensional cellular tissue including a position where the thickness is maximum (maximum point), the cell counts per area of 100 µm in the thickness direction and 50 µm in the width direction is preferably 5 or more and 70 or less, more preferably 10 or more and 60 or less, and still more preferably 15 or more and 50 or less.

In the present embodiment, the cell counts can be measured as follows using a section of the tissue cross-section in the thickness direction. In a region of the section having no void larger than a predetermined size, a position where the thickness of the three-dimensional cellular tissue is maximum (maximum point) is determined. By using a 50 µm-width strip region including the vicinity of the maximum thickness (including from the top to bottom of the tissue) as a measurement region, the cell nuclei counts in the measurement region is counted. The measurement region is determined so as not to include any voids. The predetermined size of a void refers to a maximum diameter of 50 µm or greater. The maximum diameter of a void refers to the long side when the void has a rectangular shape, the diameter when the void has a spherical shape, the major axis when the void has an elliptical shape, or the major axis of an approximate ellipse when the void has an irregular shape. Voids are not stained in an HE-stained section. However, when a region including the maximum thickness has a convex top, the tissue may be detached from the substrate. In such a case, a region near the maximum thickness and having a relatively flat top is used as the measurement region. In this case, a region with 100 µm to 650 µm width including the vicinity of the maximum thickness is used as the measurement region.

Cell nuclei at least partially included in the counted measurement region are counted as cell nuclei included in the measurement region. From the measured cell counts, the cell counts per area of 100 µm in the thickness direction and 50 µm in the width direction is calculated.

### <Second Embodiment>

The second embodiment is a method of producing three-dimensional cellular tissues which includes a first cellular tissue containing first cells and a second cellular tissue containing second cells, the second cellular tissue being in contact with the first cellular tissue, including the steps of: suspending a population of cells containing the second cells in a solution containing a cationic substance, an extracellular matrix component and a polyelectrolyte to obtain a mixture; collecting cells from the obtained mixture to obtain cell aggregates; placing the cell aggregates in contact with the first cellular tissue placed in a culture medium; and culturing the cell aggregates and the first cellular tissue without suspension to cause the cell aggregates to form the second cellular tissue, whereby a three-dimensional cellular tissue is formed.

PTL 4 shows the applicability of a three-dimensional cellular tissue having a vasculature structure created by applying the method of PTL 3 to screening applications for compounds that induce vasculature structure-specific toxicity.

As described in PTL 4, when a three-dimensional cellular tissue is formed by preparing and seeding a suspension in which all the cells that constitute the three-dimensional cellular tissue are mixed, various cells are randomly in contact with each other in the obtained structure.

However, it is reported that, in some cells such as hepatocytes, specific properties specific to the cells are expressed and maintained for a long period of time, and producing a three-dimensional cellular tissue with only that cell type is of importance (for example, see NPL 2).

Further, in evaluation of the interaction between cell type X having such properties and tissue Y formed of a different composition, for example, a stromal structure with blood vessels, it has been found that, when a three-dimensional cellular tissue formed of cell type X and tissue Y are independently and separately formed and co-cultured in the same vessel without being in contact with each other, the interaction that would be expressed when these cells are mixed and organized together may not be expressed in some cases.

The inventors of the present invention have previously conducted an experiment, in which a three-dimensional cellular tissue containing three types of cells, i.e., hepatocytes, vascular endothelial cells derived from liver sinusoids, and hepatic stellate cells was prepared by the method described in PTL 4, and exposed to compounds (for example, monocrotaline) that have been suggested to have a risk of damaging vascular endothelial cells. The results showed that reactive metabolites activated by the metabolic effects of the hepatocytes damaged the vascular endothelial cells.

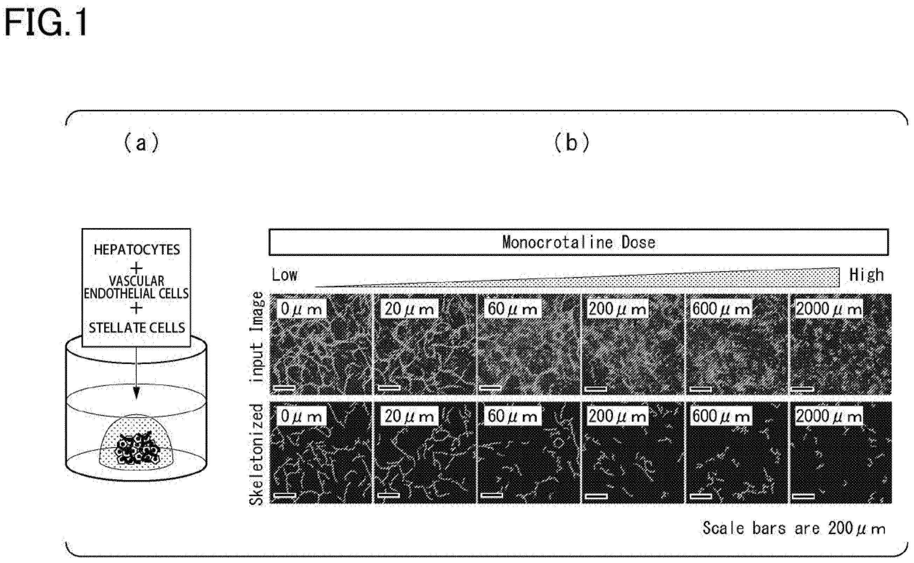

Fig. 1 (a) is a schematic diagram illustrating the above experiment. As shown in Fig. 1(a), a three-dimensional cellular tissue containing three types of cells, i.e., hepatocytes, vascular endothelial cells and hepatic stellate cells was prepared. Then, after exposure to various concentrations of monocrotaline, the three-dimensional cellular tissue was fixed and stained for CD31 by fluorescent immunostaining. CD31 is a marker for endothelial cells.

The upper row of Fig. 1(b) is a fluorescence microscopy image showing the results of immunostaining. The lower row of Fig. 1(b) is a skeletonized image obtained by processing the image in the upper row of Fig. 1B. The results shows that the vascular endothelial cells of the blood vessels were damaged in a monocrotaline concentration-dependent manner.

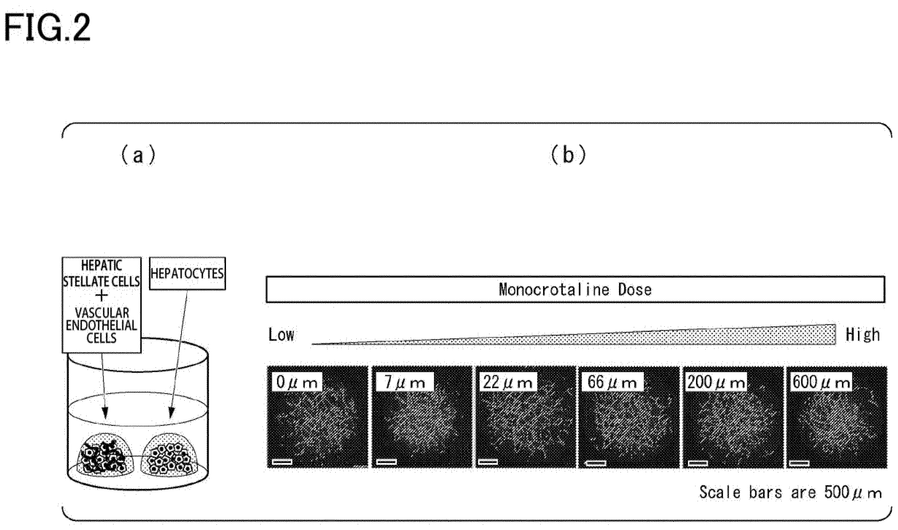

Fig. 2(a) is a schematic diagram illustrating a comparative experiment. As shown in Fig. 2(a), a liver tissue consisting only of hepatocytes and a three-dimensional cellular tissue containing two types of cells, i.e., vascular endothelial cells and hepatic stellate cells were prepared, and placed in the same well. Then, after exposure to various concentrations of monocrotaline, these tissues were fixed and stained for CD31 by fluorescent immunostaining.

Fig. 2(b) is a fluorescence microscopy image showing the results of immunostaining. The results shows that no damage to the vascular endothelial cells of the blood vessels was observed even after exposure to monocrotaline.

As seen from the above results, in order to accurately evaluate the effects of monocrotaline, it is necessary to use a three-dimensional cellular tissue in which hepatocytes and vascular tissues are located in close proximity.

However, the inventors previously found that, when a three-dimensional cellular tissue prepared by randomly mixing three types of cells as shown in Fig. 1(a) is continuously cultured, the expression of liver function-related genes decreased.

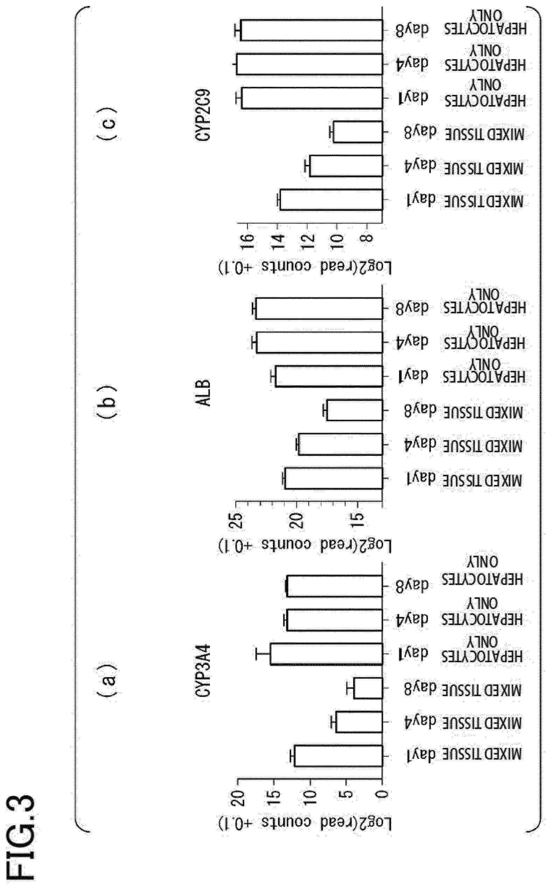

Figs. 3(a) to 3(c) are graphs showing the results of measuring the expression levels of the liver function-related genes by RNA-seq analysis after 1 day, 4 days and 8 days of culture of a three-dimensional cellular tissue prepared by randomly mixing three types of cells which are the same as those shown in Fig. 1(a), and a three-dimensional cellular tissue consisting only of hepatocytes. As the liver function-related genes, CYP3A4, ALB and CYP2C9 were examined. Fig. 3(a) is a graph showing the measurement result of CYP3A4. Fig. 3(b) is a graph showing the measurement result of ALB. Fig. 3(c) is a graph showing the measurement result of CYP2C9.

In Figs. 3(a) to 3(c), "mixed tissue" refers to the three-dimensional cellular tissue prepared by randomly mixing three types of cells which are the same as those shown in Fig. 1(a), and "hepatocytes only" refers to the three-dimensional cellular tissue consisting only of hepatocytes. The vertical axis of the graph corresponds to the relative expression level of each gene.

The results show that, the expression of liver function-related genes was maintained in the three-dimensional cellular tissue consisting only of hepatocytes, whereas the functions of the hepatocytes changed and the expression of liver function-related genes was decreased in the three-dimensional cellular tissue prepared by randomly mixing three types of cells.

This result shows that there may be cases where functions of specific cells are not accurately evaluated in the three-dimensional cellular tissue prepared by mixing a plurality of types of cells.

In view of such cases, for accurate evaluation of the interaction between cell type X and tissue Y, it is desired to use a three-dimensional cellular tissue including a three-dimensional cellular tissue in which cell type X integrated at a single location is in contact with tissue Y.

The present embodiment describes a method of producing three-dimensional cellular tissues in which a second cellular tissue is placed in contact with a first cellular tissue.

As will be described in the examples, according to the production method of the present embodiment, a three-dimensional cellular tissue in which a second cellular tissue is placed in contact with a first cellular tissue can be produced. In the three-dimensional cellular tissue obtained by the production method of the present embodiment, the second cellular tissue is integrated at a single location. Therefore, even when it is required to form a three-dimensional cellular tissue in which cell type X is integrated at a single location to maintain its function as described above referring to Figs. 1(a) to 3(c), the interaction between cell type X and tissue Y can be accurately evaluated. In this case, cell type X corresponds to the second cells.

In the production method of the present embodiment, the first cellular tissue may be a three-dimensional cellular tissue or a two-dimensional cellular tissue.

The first cellular tissue contains first cells. The first cells are cells whose interaction with the second cells is to be evaluated. The first cells may by one type of cells or may contain two or more types of cells. The first cells preferably contain stromal cells. The stromal cells refers to cells constituting supporting tissues of epithelial cells. The origin of the stromal cells are not particularly limited, and cells derived from mammals such as humans, monkeys, dogs, cats, rabbits, pigs, cows, mice and rats can be used. More specific examples of the stromal cells include fibroblasts, immune cells, vascular endothelial cells, smooth muscle cells and sinusoidal endothelial cells. Examples of the immune cells include lymphocytes, neutrophils and macrophages.

When the first cellular tissue is a three-dimensional cellular tissue, first cell aggregates may be prepared by the same method as with the cell aggregates described in the first embodiment, but not limited thereto.

The first cellular tissue is placed in a culture medium. The culture medium may be one used in the first embodiment. In addition to the above, a culture medium for hepatocytes (HCM, Lonza), a culture medium for renal cells (REGM, Lonza), a culture medium for nerve cells (AGM, Lonza), and the like may be appropriately used as the culture medium. Conditions such as the temperature and atmospheric composition of the culture environment can also be easily determined by those skilled in the art. The first cellular tissue is preferably placed in a culture vessel. Examples of the culture vessel include, but are not limited to, a dish, tube, flask, bottle, well plate, cell culture insert, and the like.

The second cells are cells whose interaction with the first cells is to be evaluated. The second cells are preferably cells required to form a three-dimensional cellular tissue while being integrated at a single location to maintain their function. The second cells may by one type of cells or may contain two or more types of cells.

For example, the first cells may contain vascular endothelial cells, and the second cells may contain hepatocytes. In this case, the interaction between the hepatocytes and the vascular endothelial cells can be accurately evaluated.

In the production method of the present embodiment, a step of suspending a population of cells containing the second cells in a solution containing a cationic substance, an extracellular matrix component and a polyelectrolyte to obtain a mixture is the same as step (A) of the three-dimensional cellular tissue described in the first embodiment. The cationic substance, the extracellular matrix component and the polyelectrolyte are the same as those described above. Further, a step of collecting cells from the mixture to obtain cell aggregates is the same as step (B) of the three-dimensional cellular tissue described in the first embodiment.

In the present embodiment, the total content of the extracellular matrix component in the mixture in step (A) described in the first embodiment is not particularly limited, and may be 0.005 mg/mL or greater and 1.5 mg/mL or less, preferably 0.005 mg/mL or greater and 1.0 mg/mL or less, more preferably 0.01 mg/mL or greater and 1.0 mg/mL or less, still more preferably 0.025 mg/mL or greater and 1.0 mg/mL or less, still more preferably 0.025 mg/mL or greater and 0.5 mg/mL or less, and still more preferably 0.025 mg/mL or greater and 0.3 mg/mL or less. The extracellular matrix component may be dissolved in an appropriate solvent before use.

In the present embodiment, the concentration of the polyelectrolyte in the mixture in step (A) described in the first embodiment is not particularly limited. Unlike the extracellular matrix component, the polyelectrolyte is effective at any concentration as long as it is below the solubility limit, and does not inhibit the effects of the extracellular matrix component. The concentration of the polyelectrolyte is preferably 0.005 mg/mL or greater, more preferably 0.005 mg/mL or greater and 1.0 mg/mL or less, still more preferably 0.01 mg/mL or greater and 1.0 mg/mL or less, still more preferably 0.025 mg/mL or greater and 1.0 mg/mL or less, and still more preferably 0.025 mg/mL or greater and 0.5 mg/mL or less.

In the production method of the present embodiment, a step of placing the cell aggregates containing the second cells in contact with the first cellular tissue placed in a culture medium is the same as step (C) of the three-dimensional cellular tissue described above. In the present embodiment, the second cell aggregates are seeded in contact with the first cellular tissue in step (C) of the above-mentioned three-dimensional cellular tissue. That is, in step (C) of the three-dimensional cellular tissue in the first embodiment, the cell aggregates are seeded in a culture medium stored in a culture vessel, whereas in the present embodiment, the second cell aggregates are placed in contact with the first cellular tissue placed in a culture medium.

Then, the first cellular tissue and the second cell aggregates are cultured, and as a result, the mixture forms a second cellular tissue, whereby a three-dimensional cellular tissue is formed as a whole. Here, the second cell aggregates are placed and then cultured without suspension to form a second cellular tissue. This facilitates the formation of a second cellular tissue integrated at a single location on the first cellular tissue.

As will be described in the examples, according to the production method of the present embodiment, a three-dimensional cellular tissue can be obtained in which the second cells integrated at a single location without dispersing are in contact with the first cellular tissue.

### [Three-Dimensional Cellular Tissue]

The present embodiment provides a three-dimensional cellular tissue containing a first cellular tissue containing first cells and a second cellular tissue containing second cells, the second cellular tissue being in contact with the first cellular tissue.

The three-dimensional cellular tissue of the present embodiment can be produced by the production method of the present embodiment described above. In the three-dimensional cellular tissue of the present embodiment, the second cellular tissue is integrated at a single location. That is, the second cellular tissue is a three-dimensional cellular tissue. Therefore, even when it is required to form a three-dimensional cellular tissue in which cell type X is integrated at a single location to maintain its function as described above referring to Figs. 1A to 3C, the interaction between cell type X and tissue Y can be accurately evaluated by using the three-dimensional cellular tissue of the present embodiment. In this case, cell type X corresponds to the second cells.

The three-dimensional cellular tissue of the present embodiment is accommodated in a culture vessel. Examples of the culture vessel include, but are not limited to, a dish, tube, flask, bottle, well plate, cell culture insert, and the like.

In the three-dimensional cellular tissue of the present embodiment, the first cells and the second cells are the same as those described above. More specifically, for example, the first cells may contain vascular endothelial cells, and the second cells may contain hepatocytes. In this case, the interaction between the hepatocytes and the vascular endothelial cells can be accurately evaluated.

### Examples

The present invention will be described in more detail and more specifically by way of examples. However, the following examples should not limit the scope of the present invention. In the following examples, collagen I was used as the collagen unless otherwise specified.

### [Experimental Example 1] Production 1 of Three-Dimensional Cellular Tissues

### <Preparation of Dispersion of Three-Dimensional Cellular Tissue>

3.0×10⁵ human hepatocytes (PXB Cells (registered trademark)) were suspended in an equal volume mixture solution (heparin/collagen solution) containing 20 µL of 1.0 mg/mL heparin/200 mM tris-HCl buffer solution (pH 7.4) and 20 µL of 0.6 mg/mL collagen/5 mM acetic acid solution (pH 3.7). After the obtained mixture was centrifuged at 400×g for 2 minutes at room temperature, the supernatant was removed. Then, the mixture was resuspended in HCM culture medium (catalog No. "CC-3198", manufactured by Lonza) containing 1 v/v% endothelial cell growth supplement (catalog No. "1052", manufactured by Sciencell, hereinafter abbreviated as ECGS) at a cell density of 1.95×10⁴/2 µL, 9.75×10³/2 µL or 4.87×10³/2 µL to thereby obtain a cell suspension. Further, a cell suspension was obtained in the same manner as above except that the cells were not suspended in a heparin/collagen solution.

### <Construction and Culture of Three-Dimensional Cellular Tissue>

2 µL of the cell suspension obtained above was seeded in a 48-well microplate in which HCM culture medium containing 500 µL of 1 v/v% ECGS was added to each well in advance, and cultured for 24 hours without suspension. Then, the culture medium was exchanged every 2 days at 500 µL per well and cultured until day 7, and then the morphology of each sample was observed using a phase contrast microscope. Fig. 1 shows the results.

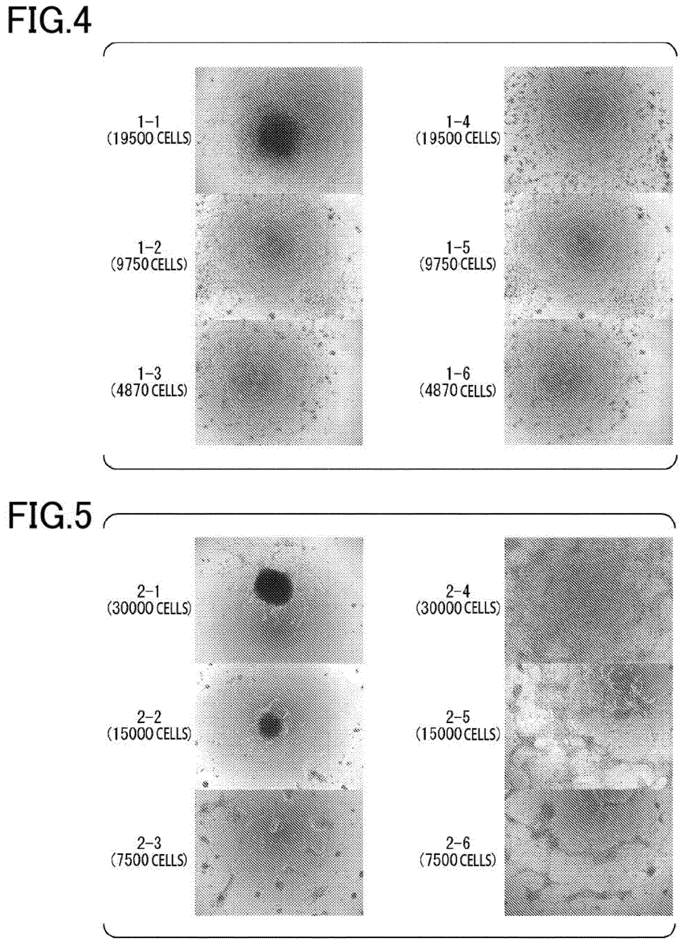

As shown in Fig. 4, in a sample (1-1) in which 1.95×10⁴ cells suspended in a heparin/collagen solution were seeded, it was observed that the cells were integrated and adhered to a single location on the culture vessel, whereby a three-dimensional cellular tissue was constructed. However, in a sample (1-2) in which 9.75×10³ cells were seeded, a sample (1-3) in which 4.87×10³ cells were seeded and samples (1-4 to 1-6) in which cells that were not suspended in a heparin/collagen solution were seeded, no three-dimensional cellular tissue was constructed.

### [Experimental Example 2] Production 2 of Three-Dimensional Cellular Tissues

### <Preparation of Dispersion of Three-Dimensional Cellular Tissue>

A cell mixture containing 65% of human hepatocytes (PXB Cells (registered trademark)), 25% of sinusoidal endothelial cells (SEC) and 10% of hepatic stellate cells (LX-2) was suspended in an equal volume mixture solution (heparin/collagen solution) containing 20 µL of 1.0 mg/mL heparin/200 mM tris-HCl buffer solution (pH 7.4) and 20 µL of 0.6 mg/mL collagen/5 mM acetic acid solution (pH 3.7). After the obtained mixture was centrifuged at 400×g for 2 minutes at room temperature, the supernatant was removed. Then, the mixture was resuspended in HCM culture medium (catalog No. "CC-3198", manufactured by Lonza) containing 1 v/v% ECGS (catalog No. "1052", manufactured by Sciencell) at a total cell density of 3.0×10⁴/2 µL, 1.5×10⁴/2 µL or 7.5×10³/2 µL to thereby obtain a mixed cell suspension. Further, a mixed cell suspension was obtained in the same manner as above except that the cell mixture was not suspended in a heparin/collagen solution.

### <Construction and Culture of Three-Dimensional Cellular Tissue>

2 µL of the mixed cell suspension obtained above was seeded in a 48-well microplate in which HCM culture medium containing 500 µL of 1 v/v% ECGS was added to each well in advance, and cultured for 24 hours without suspension. Then, the culture medium was exchanged every 2 days at 500 µL per well and cultured until day 7, and then the morphology of each sample was observed using a phase contrast microscope. Fig. 2 shows the results.

As shown in Fig. 5, in a sample (2-1) in which 3.0×10⁴ cells suspended in a heparin/collagen solution were seeded or a sample (2-2) in which 1.5×10⁴ cells suspended in a heparin/collagen solution were seeded, it was observed that the cells were integrated and adhered to a single location on the culture vessel, whereby a three-dimensional cellular tissue was constructed. However, in a sample (2-3) in which 7.5×10³ cells were seeded and samples (2-4 to 2-6) in which cells that were not suspended in a heparin/collagen solution were seeded, no three-dimensional cellular tissue was constructed.

### [Experimental Example 3] Production 3 of Three-Dimensional Cellular Tissues

### <Preparation of Dispersion of Three-Dimensional Cellular Tissue>

A cell suspension was obtained in the same manner as in Experimental Example 1 except that the cells were resuspended at a cell density of 9.75×10³/2 µL or 4.87×10³/2 µL. Further, a cell suspension in which the cells were not suspended in a heparin/collagen solution was obtained in the same manner as in Experimental Example 1.

### <Construction and Culture of Three-Dimensional Cellular Tissue>

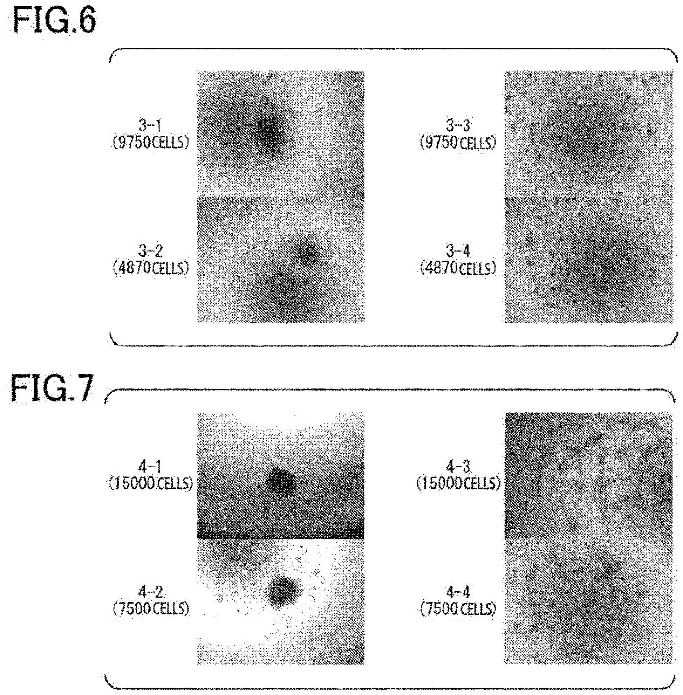

2 µL of the above cell suspension was seeded inside a liquid droplet consisting of HCM culture medium containing 30 µL of 1 v/v% ECGS formed on the surface of each well of a 48-well microplate, and cultured for 24 hours without suspension. Then, the culture medium was exchanged every 2 days at 500 µL per well and cultured until day 7, and then the morphology of each sample was observed using a phase contrast microscope. Fig. 6 shows the results.

As shown in Fig. 6, in samples (3-1, 3-2) in which cells suspended in a heparin/collagen solution were seeded, it was observed that a three-dimensional cellular tissue was constructed. However, in samples (3-3, 3-4) in which cells were not suspended in a heparin/collagen solution, the cells adhered two-dimensionally to the vessel and no three-dimensional cellular tissue was constructed.

### [Experimental Example 4] Production 4 of Three-Dimensional Cellular Tissues

### <Preparation of Dispersion of Three-Dimensional Cellular Tissue>

A mixed cell suspension was obtained in the same manner as in Experimental Example 2 except that the cells were resuspended at a cell density of 1.5×10⁴/2 µL or 7.5×10³/2 µL. Further, a cell suspension in which the cells were not suspended in a heparin/collagen solution was obtained in the same manner as in Experimental Example 2.

### <Construction and Culture of Three-Dimensional Cellular Tissue>

2 µL of the above mixed cell suspension was seeded inside a liquid droplet consisting of HCM culture medium containing 30 µL of 1 v/v% ECGS formed on the surface of each well of a 48-well microplate, and cultured for 24 hours without suspension. Then, the culture medium was exchanged every 2 days at 500 µL per well and cultured until day 7, and then the morphology of each sample was observed using a phase contrast microscope. Fig. 7 shows the results.

As shown in Fig. 7, in samples (4-1, 4-2) in which cells suspended in a heparin/collagen solution were seeded, it was observed that a three-dimensional cellular tissue was constructed. However, in samples (4-3, 4-4) in which cells that were not suspended in a heparin/collagen solution were seeded, the cells adhered two-dimensionally to the vessel and no three-dimensional cellular tissue was constructed.

### [Experimental Example 5] Production 5 of Three-Dimensional Cellular Tissues

### <Preparation of Dispersion of Three-Dimensional Cellular Tissue>

A mixed cell suspension having a total cell density of 3.0×10⁴/2 µL, 1.5×10⁴/2 µL or 7.5×10³/2 µL was obtained in the same manner as in Experimental Example 2.

### <Construction and Culture of Three-Dimensional Cellular Tissue>

Next, 2 µL of the above mixed cell suspension was seeded inside a liquid droplet consisting of HCM culture medium containing 30 µL of 1 v/v% ECGS formed on the surface of each well of a 48-well microplate, and cultured for 24 hours without suspension. Then, the culture medium was exchanged every 2 days at 500 µL per well and cultured until day 7, and then the tissue was fixed with 4% paraformaldehyde.

### <Immunohistochemical Staining>

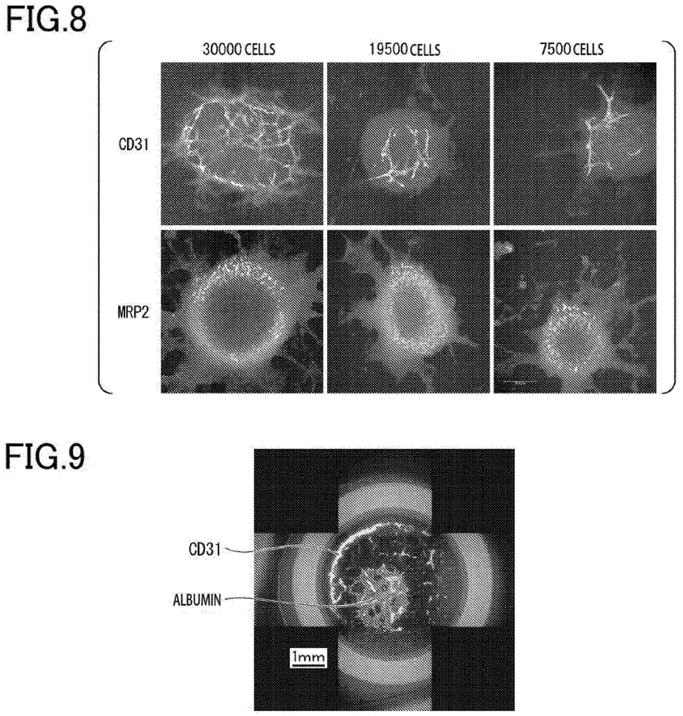

The three-dimensional cellular tissue samples obtained above were subjected to immunohistochemical staining using anti-CD31 antibody (monoclonal mouse anti-human CD31 antibody, Clone JC70A, Code M0823, manufactured by Dako) or anti-MRP2 antibody (monoclonal mouse anti-human MRP2 antibody, Clone M2 III-6, Code ab3373, manufactured by Abcam) as a primary antibody, and goat anti-mouse IgG-Alexa Fluor (registered trademark) 647 (manufactured by Thermo Fisher Scientific) as a secondary antibody. The immunohistochemical staining was performed by a known method. Fig. 8 shows the results.

As shown in Fig. 8, as a result of the immunohistochemical staining using CD31, a vasculature network was found to be formed in the tissue in all the samples. Further, as a result of the immunohistochemical staining using MRP2, bile canaliculi were found to be formed between hepatocytes in the tissue in all the samples. From the above results, the constructed samples were found to be properly organized.

### [Experimental Example 6]

### (Production 1 of Three-Dimensional Cellular Tissues)

A three-dimensional cellular tissue of Experimental Example 6 was produced. Experimental Example 6 is an example.

### <<Preparation of First Cellular Tissue>>

1.125×10⁵ hepatic stellate cells (LX-2) and 3.75×10⁴ human umbilical vein endothelial cells (HUVEC) were suspended in a culture medium to obtain a cell suspension. As the culture medium, HCM culture medium (catalog No. "CC-3198", manufactured by Lonza) to which 1 v/v% endothelial cell growth supplement (catalog No. "1052", manufactured by Sciencell, hereinafter abbreviated as "ECGS") was added was used.

Then, 100 µL/well of the above cell suspension was seeded inside the culture insert of a 96-well Transwell coated with 0.04 mg/mL fibronectin solution. Then, 300 µL/well of HCM culture medium containing 1 v/v% ECGS was added to the outside the insert, and cultured for 24 hours to form a first cellular tissue.

### <<Preparation of Second Cellular Tissue>>

3.0×10⁵ human hepatocytes (PXB Cells) were suspended in an equal volume mixture solution (heparin/collagen solution) containing 20 µL of 1.0 mg/mL heparin/200 mM tris-HCl buffer solution (pH 7.4) and 20 µL of 0.6 mg/mL collagen/5 mM acetic acid solution (pH 3.7) to obtain a mixture.

Then, the obtained mixture was centrifuged at 400×g for 2 minutes at room temperature, and the supernatant was removed to obtain a cell aggregate. HCM culture medium containing 1 v/v% ECGS was added to the cell aggregate, which was then resuspended, and the cell density was adjusted to 1.95×10⁴/2 µL to obtain a cell suspension.

Then, in a state in which 100 µL/well and 300 µL/well of HCM culture medium containing 1 v/v% ECGS were added to the inside and outside the insert, respectively, of a 96-Transwell culture insert in which the first cellular tissue was formed, 2 µL/well of the obtained cell suspension was seeded on the first cellular tissue in the insert, and cultured for 24 hours without suspension to thereby form a second cell aggregate.

Then, the culture medium was exchanged every 2 days at 100 µL/well (inside the insert) and 300 µL/well (outside the insert), and cultured until day 8 to obtain a three-dimensional cellular tissue of Example 1 containing the first cellular tissue and the second cellular tissue.

### <<Immunostaining of Three-Dimensional Cellular Tissue>>

Then, the three-dimensional cellular tissue was fixed with 4% paraformaldehyde. Then, the fixed three-dimensional cellular tissue sample was subjected to fluorescent immunostaining.

As primary antibodies, anti-CD31 antibody (monoclonal mouse anti-human CD31 antibody, Clone JC70A, Code M0823, manufactured by Dako) and anti-albumin antibody (polyclonal rabbit anti-human albumin antibody, Code 16475-1-AP, manufactured by Proteintech) were used. Further, as secondary antibodies, goat anti-mouse IgG-Alexa Fluor (registered trademark) 647 and goat anti-rabbit IgG-Alexa Fluor (registered trademark) 594 (manufactured by Thermo Fisher Scientific) were used. CD31 is a marker for endothelial cells, and albumin is a marker for hepatocytes.

Fig. 9 is an image showing the result of fluorescent immunostaining. The result shows that, in the three-dimensional cellular tissue of Experimental Example 6, the second cellular tissue was formed integrated at a single location on the first cellular tissue.

### [Experimental Example 7]

### (Production 2 of Three-Dimensional Cellular Tissues)

A three-dimensional cellular tissue of Experimental Example 7 was produced. Experimental Example 7 is an example.

### <<Preparation of First Cellular Tissue>>

A first cellular tissue was formed in a culture insert of a 96-well Transwell coated with 0.04 mg/mL fibronectin solution in the same manner as in Experimental Example 6.

### <<Preparation of Second Cellular Tissue>>

A cell suspension of human hepatocytes (PXB Cells) was obtained in the same manner as in Experimental Example 6 except that the cells were not suspended in a heparin/collagen solution.

Then, in a state in which 100 µL/well and 300 µL/well of HCM culture medium containing 1 v/v% ECGS were added to the inside and outside the insert, respectively, of a 96-Transwell culture insert in which the first cellular tissue was formed, 2 µL/well of the obtained cell suspension was seeded on the first cellular tissue in the insert, and cultured for 24 hours without suspension to thereby form a second cellular tissue.

Then, the culture medium was exchanged every 2 days at 100 µL/well (inside the insert) and 300 µL/well (outside the insert), and cultured until day 8 to obtain a three-dimensional cellular tissue of Experimental Example 7 containing the first cellular tissue and the second cellular tissue.

### <<Immunostaining of Three-Dimensional Cellular Tissue>>

Then, the three-dimensional cellular tissue of Experimental Example 7 was fixed with 4% paraformaldehyde, and subjected to fluorescent immunostaining in the same manner as in Experimental Example 6.

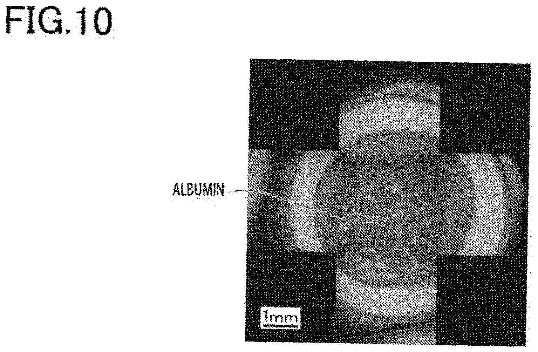

Fig. 10 is an image showing the result of fluorescent immunostaining. The result shows that, in the three-dimensional cellular tissue of Experimental Example 7, the second cellular tissue could not be integrated at a single location on the first cellular tissue, and was present in a dispersed state.

The results of Experimental Examples 6 and 7 show that, by suspending the cells constituting the second cellular tissue in a heparin/collagen solution, a three-dimensional cellular tissue can be produced in which the second cellular tissue was formed integrated at a single location on the first cellular tissue.

### [Industrial Applicability]

By using the method of the present invention, the number of cells to be seeded to produce a three-dimensional cellular tissue can be reduced, and a technique for producing integrated three-dimensional cellular tissue at a single location can be provided.

## Claims

1. A method of culturing three-dimensional cellular tissues, comprising the steps of:
mixing cells with a cationic substance, an extracellular matrix component and a polyelectrolyte to obtain a mixture;
collecting cells from the obtained mixture to obtain cell aggregates;
seeding the cell aggregates in a culture medium stored in a culture vessel; and
culturing the seeded cell aggregates without suspension to thereby obtain a three-dimensional cellular tissue.

2. The method of culturing three-dimensional cellular tissues according to claim 1, wherein
in the step of seeding the cell aggregates in a culture medium stored in a culture vessel, 3×10⁴ to 1×10⁷ cells are seeded per mL of culture medium.

3. The method of culturing three-dimensional cellular tissues according to claim 1 or 2, wherein
the culture vessel is an adhesive culture vessel.

4. The method of culturing three-dimensional cellular tissues according to claim 1 or 2, wherein
the extracellular matrix component is selected from the group consisting of collagen, laminin, fibronectin, vitronectin, elastin, tenascin, entactin, fibrillin, proteoglycan, and combinations thereof, and
the polyelectrolyte is selected from the group consisting of glycosaminoglycans, dextran sulfate, rhamnan sulfate, fucoidan, carrageenan, polystyrene sulfonic acid, polyacrylamido-2-methylpropanesulfonic acid, polyacrylic acid, and combinations thereof.

5. The method of culturing three-dimensional cellular tissues according to claim 1 or 2, wherein
a concentration of the extracellular matrix component in the mixture is 0.01 mg/mL or greater and less than 1.0 mg/mL, and
a concentration of the polyelectrolyte in the mixture is 0.01 mg/mL or greater and less than 10 mg/mL.

6. A method of producing three-dimensional cellular tissues, comprising the step of:
suspending a population of cells containing second cells in a solution containing a cationic substance, an extracellular matrix component and a polyelectrolyte to obtain a mixture;
collecting cells from the obtained mixture to obtain cell aggregates;
placing the cell aggregates in contact with a first cellular tissue containing first cells placed in a culture medium; and
culturing the cell aggregates and the first cellular tissue without suspension to cause the cell aggregates to form a second cellular tissue, whereby a three-dimensional cellular tissue is formed, in which the second cellular tissue is in contact with the first cellular tissue.

7. The method of producing three-dimensional cellular tissues according to claim 6, wherein
the first cells contain vascular endothelial cells, and the second cells contain hepatocytes.

8. A three-dimensional cellular tissue containing a first cellular tissue containing first cells and a second cellular tissue containing second cells, the second cellular tissue being in contact with the first cellular tissue.

9. The three-dimensional cellular tissue according to claim 8, wherein
the first cells contain vascular endothelial cells, and the second cells contain hepatocytes.
